# EUROPÄISCHE PATENTSCHRIFT

(11) **EP 2 926 733 B1**
(45) Veröffentlichungstag und Bekanntmachung des Hinweises auf die Patenterteilung: **02.11.2016**
(21) Anmeldenummer: 15157272.4
(22) Anmeldetag: 03.03.2015
(51) Int. Cl.: A61B 6/00, G06T 19/00, G02B 27/01, G06T 7/00, G01S 5/00, H04N 13/00, H04N 13/02, H04N 13/04

(54) **Triangulationsbasierte Tiefen- und Oberflächen-Visualisierung**
Triangulation-based depth and surface visualisation
Visualisation de surface et de profondeur basée sur la triangulation

(30) Priorität: 31.03.2014 DE 102014206004
(43) Veröffentlichungstag der Anmeldung: 07.10.2015
(73) Patentinhaber: Siemens Healthcare GmbH, 91052 Erlangen (DE)
(72) Erfinder: Grafenberg, Alexander, 91090 Effeltrich (DE)

(56) Entgegenhaltungen:
- US-A- 5 091 926
- US-A1- 2009 034 820
- US-B1- 6 608 884
- TOBIAS SIELHORST ET AL: "Advanced Medical Displays: A Literature Review of Augmented Reality", JOURNAL OF DISPLAY TECHNOLOGY, IEEE SERVICE CENTER, NEW YORK, NY, US, Bd. 4, Nr. 4, 2. Dezember 2008 (2008-12-02), Seiten 451-467, XP011237912, ISSN: 1551-319X, DOI: 10.1109/JDT.2008.2001575
- TERRY M PETERS: "TOPICAL REVIEW; Image-guidance for surgical procedures", PHYSICS IN MEDICINE AND BIOLOGY, INSTITUTE OF PHYSICS PUBLISHING, BRISTOL GB, Bd. 51, Nr. 14, 21. Juli 2006 (2006-07-21) , Seiten R505-R540, XP020095863, ISSN: 0031-9155, DOI: 10.1088/0031-9155/51/14/R01

## Beschreibung

Die vorliegende Erfindung liegt auf dem Gebiet der intraoperativen Visualisierungstechniken zur Visualisierung von medizinischen Strukturen (z.B. Knochen, Organen oder Implantaten) im Körperinneren des Patienten und somit "in der Tiefe", insbesondere mit Röntgen-basierten Verfahren und zur Visualisierung von Oberflächenstrukturen der Körperoberfläche. Die Erfindung kann vorzugsweise bei intraoperativen Eingriffen angewendet werden.

Im Stand der Technik sind Navigationssysteme bekannt, um den Arzt bei der Durchführung einer medizinischen Intervention gezielt im Raum leiten zu können. Dazu ist es jedoch notwendig, zusätzlich zu der bildgebenden Einrichtung noch weitere Module bereitzustellen, die im Rahmen des Navigationssystems erforderlich sind. Dies betrifft insbesondere das Bereitstellen von separaten Kameras mit entsprechenden Versorgungsleitungen und Netzwerkverbindungen. Diese können jedoch die Beweglichkeit des Arztes während des Eingriffs nachteilig einschränken und führen nicht selten zu deutlichem Zeitverlust. Ebenso ungünstig hat sich die Verwendung von Markern (z.B. Infrarot oder passiven Markern) erwiesen; da auch diese erst installiert und mit weiteren Geräten ausgelesen werden müssen. Deshalb ist es wünschenswert, ein Visualisierungssystem bereitstellen zu können, das ohne die Verwendung von zusätzlichen Bauteilen (wie separaten Kameras und einem Markersystem) auskommt.

Die Anwendung bzw. Applikation von strukturiertem Licht auf Oberflächen zur Erfassung der dreidimensionalen Form der Oberfläche ist bekannt. Dazu wird ein Lichtmuster auf ein Objekt, also hier: den Patienten oder ein Körperteil desselben, projiziert und die Musterverformung wird mittels einer Kamera beobachtet. Dabei ist sozusagen die geometrische (dreidimensionale) Oberflächenstruktur des Patientenkörpers im Licht codiert und wird damit erfassbar.

Aus dem Forschungsbereich der Virtuellen Realität ist es auch bekannt, kopfgetragene Visualisierungseinrichtungen, so genannte Head-Mounted Displays (kurz: HMD) zu verwenden. Dies sind Brillen, deren Gläser sowohl zur Durchsicht als auch als Projektionsfläche für digitale Bilder geschaltet und verwendet werden können. Beispiele für HMD-Einrichtungen sind sowohl in US2013/0021373A1 als auch in Advanced Medical Displays: A Literature Review of Augmented Reality, T. Sielhorst et. al., Journal of Display Technology, 4(4):2008 beschrieben.

Im medizinischen Bereich ist es jedoch wichtig, dass der einen Eingriff durchführende Chirurg oder der Arzt nicht unnötig von der relevanten Körperstruktur weg sehen muss, um weitere notwendige Informationen über die zu untersuchende Körperstruktur (z.B. Röntgenbild) zu erhalten. So ist es beispielsweise sehr hinderlich, dass der Arzt von dem Operationsfeld weg sehen muss, um ein Röntgenbild zu analysieren, das auf einem separaten Monitor angezeigt wird.

In der Medizintechnik fehlen gegenwärtig Systeme, die eine bildliche Oberflächeninformation in dreidimensionaler Form und bildliche Tiefeninformation von einem Körpersegment in geeigneter Weise kombinieren und ortsaufgelöst fusionieren. Die Erfindung hat sich deshalb zur Aufgabe gemacht, ein Visualisierungssystem bereitzustellen, das die Nachteile aus dem Stand der Technik überwindet. Das Visualisierungssystem soll ohne Zusatzaufwand seitens des Arztes oder des Anwenders im Operationssaal ausführbar sein. Des Weiteren soll die Zeit und die Qualität, mit der jeweils die bereitgestellten Informationen verfügbar sind, verbessert werden.

Diese Aufgabe wird durch die beiliegenden nebengeordneten Patentansprüche gelöst, insbesondere durch ein Visualisierungssystem und Verfahren und ein Steuerprogramm.

Nachstehend wird die Lösung der Aufgabe in Bezug auf die beanspruchte Vorrichtung bzw. das System beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf ein System gerichtet sind) auch mit den Merkmalen, die in Zusammenhang mit dem Verfahren beschrieben oder beansprucht sind, weitergebildet sein und umgekehrt. Die entsprechenden funktionalen Merkmale des Verfahrens werden dabei durch entsprechende gegenständliche Module, insbesondere durch Microprozessor- oder Hardware-Module, ausgebildet.

Gemäß einem Aspekt betrifft die Erfindung ein Visualisierungssystem zur Visualisierung von dreidimensionalen Strukturen im Rahmen einer Intervention an einem Patienten mittels einer bildgebenden Einrichtung, z.B. mit einem C-Bogen, umfassend:
- Ein C-Bogen-Projektionsmodul, das dazu bestimmt ist, strukturiertes Licht auf einen ersten ausgewählten Bereich einer Körperoberfläche des Patienten zu projizieren, wobei das C-Bogen-Projektionsmodul in der bildgebenden Einrichtung integriert ist und während sich die bildgebende Einrichtung zur Bildakquisition bewegt
- Eine Visualisierungsbrille, die ein HMD-Projektionsmodul umfasst, das dazu bestimmt ist, auf ein Startsignal hin strukturiertes Licht auf einen zweiten ausgewählten Bereich einer Körperoberfläche des Patienten zu projizieren
- Ein Kamerasystem, das dazu bestimmt ist, das von dem C-Bogen-Projektionsmodul und das von dem HMD-Projektionsmodul projizierte strukturierte Licht zu erfassen, um mit einem Prozessor zumindest einen digitalen Bilddatensatz einer 3D-Oberflächenstruktur zu berechnen, und wobei das Kamerasystem in der bildgebenden Einrichtung und/oder in der Visualisierungsbrille integriert ist
- Eine Synchronisierungseinrichtung, die dazu bestimmt ist, ein Triggersignal auszugeben, wenn sie automatisch erfasst, dass der vom C-Bogen-Projektionsmodul projizierte erste ausgewählte Bereich mit dem vom HMD-Projektionsmodul projizierte zweite ausgewählte Bereich übereinstimmen bzw., dass die mit dem Kamerasystem erfassten jeweiligen digitalen Bilddatensätze übereinstimmen
- Eine Fusionseinheit, die dazu bestimmt ist, in Antwort auf das Triggersignal der Synchronisierungseinheit, einen kombinierten Bilddatensatz zu erzeugen und auf der Visualisierungsbrille auszugeben, wobei der kombinierte Bilddatensatz das von der bildgebenden Einrichtung erfasste Bild und den digitalen Bilddatensatz des Kamerasystems der Körperoberfläche korreliert.

Im Folgenden werden die im Rahmen dieser Anmeldung verwendeten Begrifflichkeiten näher erläutert.

Das Visualisierungssystem umfasst mehrere physikalische Bauteile und wird vorzugsweise intraoperativ, also während eines chirurgischen Eingriffs verwendet. Alternativ kann es auch bei der Erfassung von Bilddaten ohne chirurgischen Eingriff verwendet werden, wie z.B. bei der Planung desselben oder bei einer rein bildgebenden Maßnahme zur Untersuchung eines Patienten. Das Visualisierungssystem ist markerlos, das heisst, es verwendet keine und erfordert auch nicht den Einsatz von Markern, um Orts- oder Bewegungsdaten zu erfassen. Dies stellt einen wichtigen Vorteil gegenüber bekannten Systemen dar, da Marker nicht positioniert und gewartet werden müssen. Insbesondere muss während des Eingriffs darauf geachtet werden, dass die Markersignale auch ungestört empfangen werden können und nicht etwa durch weitere medizinische Gegenstände im Raum unterbrochen werden.

"Strukturen" können Organe oder Körpersegmente im Patientenkörper sein oder künstliche medizinische Objekte, wie Implantate oder chirurgisches Material, wie Schrauben, Stifte oder dergleichen.

Die bildgebende Einrichtung ist üblicherweise ein beweglicher bzw. mobiler C-Bogen, der zur Erfassung von Röntgenbildern bestimmt ist. Selbstverständlich kann die vorliegende Erfindung auch auf Tomosyntheseeinrichtungen und andere bildgebende Modalitäten angewendet werden, wie z.B. MRT-Anlagen, PET-Einrichtungen etc.. Gemäß einem Aspekt der Erfindung ist die bildgebende Einrichtung zur Bilddatenakquisition von Strukturen bestimmt, die sich nicht auf einer Körperoberfläche des Patienten, sondern im Inneren des Patienten befinden.

Das C-Bogen-Projektionsmodul ist eine Einrichtung zur Projektion von strukturiertem Licht, wie z.B. einem Lichtmuster oder Licht in unterschiedlichen Wellenlängen bzw. Farben. Wesentlich ist, dass das C-Bogen-Projektionsmodul direkt in den C-Bogen bzw. in die bildgebende Einrichtung integriert ist. Dabei ist es an einer vorbestimmten und damit bekannten Position an den C-Bogen befestigt. Diese Position kann dann für spätere Orts- Bewegungs- und/oder Bildberechnungen verwendet werden. Vorzugsweise kann das C-Bogen-Projektionsmodul wiederlösbar aber fest an dem C-Bogen befestigt sein.

Neben dem C-Bogen weist auch die Visualisierungsbrille ein solches Projektionsmodul auf, nämlich das HMD-Projektionsmodul. Es ist ebenfalls zur Projektion von strukturiertem Licht bestimmt. Insbesondere können beide Projektionsmodule von derselben Steuereinheit gesteuert werden und dieselbe Art von strukturiertem Licht applizieren. Die Steuereinheit kann gemäß einer Ausführung in einem Prozessor integriert sein. Sie kann ferner auch die zeitliche Ausführung der Lichtprojektion steuern, insbesondere auch in Relation zwischen den beiden Projektionsmodulen.

Die Visualisierungsbrille fungiert als kopfgetragene Blickfeldeinrichtung und kann auch als Head-Mounted-Display bezeichnet werden. Variationen sind denkbar. So kann die Brille auch als Videobrille, Helmdisplay oder nur als Okular mit einem Brillenglas oder mit einer Linse ausgebildet sein.

Das Kamerasystem kann eine oder zumindest zwei Kameras, vorzugsweise CCD-Kameras umfassen. Das Kamerasystem ist dazu bestimmt, das applizierte strukturierte Licht zu erfassen. Soll farbiges Licht appliziert werden, muss es sich entsprechend um eine Farbkamera handeln, ansonsten genügt eine S/W-Erfassung. Vorzugsweise sind zwei Kameras vorgesehen: eine ist in dem C-Bogen integriert (z.B. auch als abnehmbares aber fest integriertes Modul) und eine zweite ist in der Visualisierungsbrille integriert. Das Kamerasystem umfasst einen Prozessor oder steht mit einem separaten Prozessor in Datenaustausch (vorzugsweise über eine drahtlose Funkverbindung). Der Prozessor ist eine Verarbeitungseinheit zur computerbasierten und automatischen Verarbeitung von digitalen Datensätzen, die von dem Kamerasystem erfasst worden sind. Insbesondere erzeugt der Prozessor einen digitalen Bilddatensatz. Zusätzlich kann der Prozessor noch weitere Bildverarbeitungen durchführen, z.B. Filterfunktionen, Speichern der Daten, Vergleich mit SOLL-Werten etc.. Gemäß einem Aspekt der Erfindung ist das Kamerasystem zur Erfassung von dreidimensionalen Oberflächenstrukturen bestimmt, die sich nicht im Inneren des Patienten, sondern auf einer Körperoberfläche des Patienten befinden.

Die Synchronisierungseinrichtung ist ein elektronisches Bauteil. Es dient als computer-basiertes Modul zum Abgleich der von den beiden Kameras erfassten Bilddaten. Insbesondere erfolgt ein Vergleich auf Identität bzw. Übereinstimmung in einem voreinstellbaren Toleranzbereich mit sehr hoher Genauigkeit. Dieser Identitätsvergleich dient dazu, den C-Bogen und die Visualisierungsbrille zu synchronisieren. Damit wird sichergestellt, dass das Projektionsmodul des C-Bogens und das HMD-Projektionsmodul der Visualisierungsbrille Licht auf exakt denselben Körperbereich des Patienten applizieren. Nur in diesem Fall wird ein Triggersignal ausgegeben.

Auf das Triggersignal hin kann die Fusionseinheit aktiviert werden. Die Fusionseinheit ist ebenfalls ein elektronisches Bauteil und dient zur Erzeugung eines kombinierten Bilddatensatzes. Der kombinierte Bilddatensatz umfasst ein Tiefenbild (erfasst von der bildgebenden Einrichtung, z.B. Röntgen) und ein Oberflächenbild (erfasst von dem Kamerasystem oder einer Kamera des Kamerasystems von der Körperoberfläche). Dabei sind das Tiefenbild und das Oberflächenbild ortskorreliert. Das Tiefenbild betrifft dieselbe Körperstruktur wie das Oberflächenbild und umgekehrt. Der kombinierte Bilddatensatz wird auf die Visualisierungsbrille projiziert. Der Betrachter erhält somit drei unterschiedliche Bilddatensätze von ein und derselben Körperstruktur zeitgleich und in ein und derselben physikalischen Blickachse:
1. Den von der bildgebenden Einrichtung erfassten Bilddatensatz (z.B. Röntgenbild)
2. Das Oberflächenbild in dreidimensionaler Form, wobei der Inhalt des Oberflächenbildes mit dem Inhalt des ersten Bilddatensatzes übereinstimmt und
3. Das reine Bild beim Betrachten der Körperstruktur (durch die Brille ohne Brillenprojektion oder beim direkten Betrachten der Körperstruktur).

Gemäß einer bevorzugten Ausführungsform ist die Synchronisierungseinrichtung in die Visualisierungsbrille integriert. Dasselbe gilt für die Fusionseinheit. Alternativ können die vorstehend genannten elektronischen Bauteile auch in den Prozessor oder in ein über eine Netzwerkverbindung angeschlossenes weiteres Verarbeitungsmodul integriert sein und somit außerhalb der Visualisierungsbrille angeordnet sein.

Gemäß einer weiteren Ausführungsform sind die Synchronisierungseinrichtung und die Fusionseinheit in einem Modul integriert.

Gemäß einer weiteren Ausführungsform berechnet der Prozessor den vom Kamerasystem erfassten digitalen Bilddatensatz auch während einer Bewegung, insbesondere einer rotatorischen und/oder translatorischen Bewegung der bildgebenden Einrichtung, indem ein automatisches, computer-basiertes Verfahren zur optischen Triangulation angewendet wird. Dieses Verfahren kommt auch zum Einsatz, wenn der Arzt seine Kopfhaltung mit der Visualisierungsbrille verändert also während einer Bewegung des HMD-Projektionsmoduls der Visualisierungsbrille. Zusätzlich wird automatisch über einen Sensor der Abstand der jeweiligen Kamera von dem Objekt (hier: Oberfläche) erfasst oder von einem Speicherort eingelesen, falls diese bekannt ist. Dabei bezeichnet die Bewegungsparallaxe die scheinbar relative Versetzung bzw. Bewegung zwischen Objekten, die sich in unterschiedlicher Entfernung vom optischen Erfassungssystem bzw. Betrachter (hier: Kamera) befinden, hervorgerufen durch eine Veränderung des Betrachterstandpunktes oder durch die Bewegung der Objekte relativ zum Betrachter. Entferntere Objekte bewegen sich dabei scheinbar langsamer als Objekte die einen geringeren Abstand vom Betrachter aufweisen. Die Bewegungsparallaxe umfasst somit einen Tiefenhinweis, der bei der Berechnung in dem Prozessor und/oder in der Fusionseinheit berücksichtigt wird.

Eine weitere Aufgabenlösung besteht in einem Visualisierungsverfahren zur Visualisierung von dreidimensionalen Strukturen im Rahmen einer Intervention an einem Patienten mittels einer bildgebenden Einrichtung, umfassend folgende Verfahrensschritte:
- Erfassen eines Bilddatensatzes mit der bildgebenden Einrichtung, insbesondere mit einem C-Bogen
- Erstes Projizieren von strukturiertem Licht auf einen ersten ausgewählten Bereich einer Körperoberfläche des Patienten mit einem C-Bogen-Projektionsmodul, das in der bildgebenden Einrichtung integriert ist, während sich die bildgebende Einrichtung zum Zwecke der Bildakquisition bewegt
- Zweites Projizieren von strukturiertem Licht auf einen zweiten ausgewählten Bereich der Körperoberfläche des Patienten mit einem HMD-Projektionsmodul, das in einer Visualisierungsbrille integriert ist,
- Erfassen zumindest eines digitalen Bilddatensatzes mit dem projizierten ersten und/oder zweiten ausgewählten Bereich zum Berechnen einer 3D-Oberflächenstruktur in dem jeweils projizierten Bereich
- Ausgeben eines Triggersignals, falls automatisch erfasst wird, dass der erste ausgewählte Bereich und der zweite ausgewählten Bereich übereinstimmen
- In Antwort auf das Triggersignal: Erzeugen eines kombinierten Bilddatensatzes und Ausgeben desselben auf der Visualisierungsbrille, wobei der kombinierte Bilddatensatz berechnet wird, indem das von der bildgebenden Einrichtung erfasste Bild mit dem zumindest einen digitalen Bilddatensatz mit der berechneten 3D-Oberflächenstruktur korreliert wird.

Andere Ausführungen der Erfindung sehen hier abweichende Reihenfolgen der vorstehenden Bearbeitungsschritte vor. So kann beispielweise auch in einem ersten Schritt das erste Projizieren durch ausgeführt werden und erst nachfolgend die Röntgenaufnahme erfasst werden.

Gemäß einem Aspekt werden das erste Projizieren und das zweite Projizieren zeitgleich oder in einem übereinstimmenden Zeitbereich ausgeführt (also zumindest mit zeitlicher Überlappung; es kann aber unterschiedliche Anfangszeitpunkte geben) .

Gemäß einem Aspekt werden das Erfassen des zumindest einen digitalen Bilddatensatzes ausgeführt, wenn (zeitgleich oder nachdem) das zweite Projizieren ausgeführt wird. Der Begriff "wenn" kann hier als zeitliche Bedingung oder auch als kausale Bedingung verstanden werden. Dies bedeutet, dass das Erfassen nur dann ausgeführt, wenn das Projizieren ausgeführt ist oder wird. Ebenso kann das Erfassen in dem Zeitbereich ausgeführt werden, in dem auch das Projizieren erfolgt.

Gemäß einem Aspekt wird das Erfassen des zumindest einen digitalen Bilddatensatzes zeitgleich oder in einem übereinstimmenden Zeitbereich ausgeführt wie die Bildakquisition mittels der bildgebenden Einrichtung.

Die vorstehend beschriebenen, erfindungsgemäßen Ausführungsformen des Verfahrens können auch als Computerprogrammprodukt mit einem Computerprogramm ausgebildet sein, wobei der Computer zur Durchführung des oben beschriebenen, erfindungsgemäßen Verfahrens veranlasst wird, wenn das Computerprogramm auf dem Computer bzw. auf einem Prozessor des Computers ausgeführt wird.

Eine alternative Aufgabenlösung besteht auch in einem Computerprogramm mit Computer-Programmcode zur Durchführung aller Verfahrensschritte des beanspruchten oder oben beschriebenen Verfahrens, wenn das Computerprogramm auf dem Computer ausgeführt wird. Dabei kann das Computerprogramm auch auf einem maschinenlesbaren Speichermedium gespeichert sein.

Eine alternative Aufgabenlösung sieht ein Speichermedium vor, das zur Speicherung des vorstehend beschriebenen, computerimplementierten Verfahrens bestimmt ist und von einem Computer lesbar ist.

Es liegt im Rahmen der Erfindung, dass nicht alle Schritte des Verfahrens zwangsläufig auf ein und derselben Computerinstanz ausgeführt werden müssen, sondern sie können auch auf unterschiedlichen Computerinstanzen (umfassend Rechner, Prozessor oder weitere computer-basierte automatische Verarbeitungsinstanzen) ausgeführt werden. Auch kann die Abfolge der Verfahrensschritte, wie vorstehend erläutert, gegebenenfalls variiert werden.

Darüber hinaus ist es möglich, dass einzelne Abschnitte des vorstehend beschriebenen Verfahrens in einer verkaufsfähigen Einheit und die restlichen Komponenten in einer anderen verkaufsfähigen Einheit - sozusagen als verteiltes System - ausgeführt werden können.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Figur 1: eine übersichtsartige Darstellung eines Gesamtaufbaus einer bevorzugten Ausführungsform des erfindungsgemäßen Visualisierungssystems,
- Figur 2: eine schematische Übersichtsdarstellung über eine Visualisierungsbrille,
- Figur 3: eine schematische Übersichtsdarstellung über eine Prozessor zur Berechnung eines kombinierten Bilddatensatzes und
- Figur 4: einen Datenfluss eines Verfahrens entsprechend einer bevorzugten Ausführungsform der Erfindung.

Im Folgenden wird die Erfindung näher unter Bezugnahme auf die Figuren erläutert.

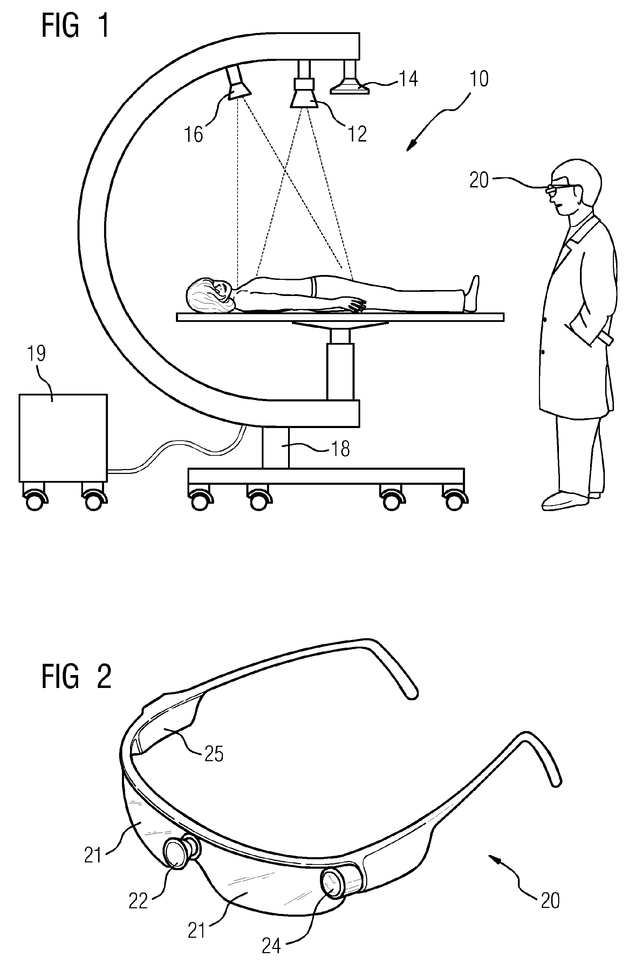

**Figur 1** zeigt auf schematische Weise ein Visualisierungssystem, das insbesondere während einer medizinischen Intervention eingesetzt wird. Dabei kann es sich um einen chirurgischen Eingriff oder um eine reine Bilddatenerfassung zu Planung von weiteren Schritten oder um weitere Maßnahmen handeln. Das Visualisierungssystem dient zur Visualisierung beziehungsweise Darstellung von 3-dimensionalen Patientenstrukturen im Raum. Dabei sollen sowohl in einem vorwählbaren Bereich (region of interest - ROI) eine 3-dimensionales Abbild der Körperoberfläche erhalten werden und es soll das mit einer bildgebenden Einrichtung 10 erfasste Bild dargestellt werden. Bei der bildgebenden Einrichtung handelt es sich insbesondere um einen mobilen C-Bogen, der mobil und beispielsweise auf einem Rollwagen 18 gelagert sein kann. Der C-Bogen dient zur Erfassung von Röntgenbildern und/oder Tomosynthesebildern. Selbstverständlich kann die Erfindung auch auf andere bildgebende Modalitäten angewendet werden, wie zum Beispiel Magnetresonanztomographen. Die folgende Figurenbeschreibung beschäftigt sich jedoch hauptsächlich mit einem Röntgen-C-Bogen 10, da dies eine bevorzugte Ausführungsform der Erfindung ist, ohne jedoch den Schutzumfang der Erfindung auf diese Ausführung zu beschränken.

Das Visualisierungssystem dient somit zur gleichzeitigen Darstellung von der Körperoberfläche und von Tiefenbildern des Patienten in einem interessierenden Bereich (ROI). Wichtig ist, dass der Arzt beispielsweise während eines Eingriffs gleichzeitig Informationen über die Körperoberfläche erhält und zugeordnete Bildinformationen zu den unter der Körperoberfläche liegenden ("inneren" oder "Tiefen-") Strukturen, wie beispielsweise Knochen, Organen, Gefäßen, Implantaten etc. Die zuletzt genannten Strukturen können mittels des C-Bogens 10 akquiriert werden. Das erfindungsgemäße Visualisierungssystem korreliert somit die Körperoberflächenstrukturen mit den Körpertiefenstrukturen und zeigt diese auf einer Visualisierungsbrille 20 an.

Das Visualisierungssystem ist markerlos und erfordert keine Platzierung von im Stand der Technik bekannten (aktiven oder passiven) Markern.

Das Visualisierungssystem ist in Figur 1 schematisch dargestellt und umfasst den C-Bogen 10 und eine Visualisierungsbrille 20, die brillenartig ausgebildet sein kann und vom Arzt somit während des Eingriffs auf dem Kopf getragen werden kann. Die Visualisierungsbrille 20 kann auch als kopfgetragene Blickfeldeinrichtung oder sogenanntes Head-Mounted Display bezeichnet werden. Die Visualisierungsbrille 20 kann somit auch in der Art eines Kopfhelms oder als Blickfeldeinrichtung ausgestaltet sein, die lediglich ein Brillenglas 21 aufweist. Bevorzugt ist jedoch eine Visualisierungsbrille 20, die zwei Brillengläser 21 umfasst.

Der C-Bogen 10 umfasst, wie im Stand der Technik auch bekannt, zumindest eine Röntgenquelle 12. Der Begriff "Röntgenquelle" ist in diesem Zusammenhang nicht einschränkend zu verstehen und soll ein Röntgensystem aus Röntgenemittern kennzeichnen. Üblicherweise kann eine Röntgenquelle aus einer Vielzahl von unterschiedlich angeordneten Röntgenemittern bestehen, die darüber hinaus noch innerhalb des C-Bogens 10 beweglich sind (zum Beispiel schwenkbar gelagert). Als Gegenstück und zur Erfassung der den Patienten beziehungsweise den relevanten Bereich des Patienten durchdringenden Röntgenstrahlung ist in dem C-Bogen 10 ein Röntgendetektor vorgesehen, der in Figur 1 nicht dargestellt ist, weil er zum Verständnis der Erfindung unerheblich ist. Der C-Bogen 10 umfasst erfindungsgemäß neben den bekannten Bauteilen noch weitere physikalische Bauteile:
1.ein C-Bogen-Projektionsmodul 16 und
2.eine fest positionierte Kamera 14.

Das C-Bogen-Projektionsmodul 16 und die Kamera 14 sind fest in den C-Bogen 10 integriert. Der Begriff "fest" bedeutet in diesem Zusammenhang, dass beide physikalische Bauteile eine feste Position haben, die für spätere Berechnungen verwendet werden kann. Dennoch können die beiden Bauteile oder eines der Bauteile abnehmbar an den C-Bogen 10 befestigt werden, etwa durch Anklicken oder Einrasten. Wesentlich ist jedoch, dass sowohl das C-Bogen-Projektionsmodul 16 als auch die Kamera 14 eine jeweils zugeordnete definierte Position insbesondere in Bezug auf die Röntgenquelle 12 haben. Der C-Bogen 10 bewegt sich während der Bildakquisition um den Patienten (Schwenkbewegung) und kann auch translatorisch bewegt werden. Die erfassten Röntgenbilder werden üblicherweise an einen über eine entsprechende Netzwerkverbindung angeschlossenen Rechner 19 zum Post-Processing beziehungsweise zur weiteren Verarbeitung weitergeleitet. Der Rechner 19 kann jedoch auch teilweise oder vollständig direkt in den C-Bogen integriert sein. Darüber hinaus kann der Rechner 19 auch als separates Modul bereitgestellt werden. Es liegt ebenso im Rahmen der Erfindung, hier mehrere Prozessoren beziehungsweise Rechner oder Rechnereinheiten vorzusehen, die insgesamt als Rechner 19 bezeichnet werden. Die einzelnen Bauteile beziehungsweise Einheiten können auch verteilt angeordnet sein.

**Figur 2** zeigt übersichtsartig und auf schematische Weise eine Visualisierungsbrille, die in den Figuren grundsätzlich mit dem Bezugszeichen 20 gekennzeichnet ist. Die Visualisierungsbrille 20 umfasst ein HMD-Projektionsmodul 22, eine Kamera 24 und einen Prozessor 25. Diese Bauteile werden vorzugsweise gemeinsam mit den beiden Brillengläsern 21 von einem Gestell getragen. Das HMD-Projektionsmodul 22 dient zur Projektion von strukturiertem Licht auf einen zweiten ausgewählten Bereich einer Körperoberfläche des Patienten. Gemäß einer bevorzugten Ausführungsform ist es vorgesehen, dass das HMD-Projektionsmodul 22 erst nach Erfassen eines Startsignals s aktiviert wird. Mit anderen Worten verbleibt das HMD-Projektionsmodul 22 inaktiv und sendet keine Lichtstrahlung, falls kein entsprechendes Startsignal s empfangen werden kann. Das von dem HMD-Projektionsmodul 22 projizierte Licht kann ein einfaches Lichtmuster sein (zum Beispiel Streifenmuster) oder ein Farbmuster sein, das auf eine 3-dimensionale Oberfläche appliziert wird. Aufgrund einer Triangulationsberechnung kann durch die 3-dimensionale Form verursachte Verzerrung des Lichtmusters von einer Kamera, z. B. der Kamera 24 der Visualisierungsbrille 20, erfasst und von einem Prozessor 25 berechnet werden, welches die zugehörige Bilddaten der 3D-Oberflächenstruktur sind. Somit können die Bilddaten der 3D-Oberflächenstruktur berechnet und visualisiert werden. Zur Erfassung des projizierten Lichtmusters ist die Kamera 24 vorgesehen. Alternative Ausführungsformen sehen hier eine andere Kamera vor, die z. B. im C-Bogen 10 angeordnet sein kann oder es können auch andere Kameras bereitgestellt werden, die ohnehin im Rahmen des intraoperativen Eingriffs um das OP-Feld angeordnet sind.

Der Prozessor 25 der Visualisierungsbrille 20 dient zum Generieren eines digitalen Bilddatensatzes von der 3-dimensionalen Körperoberfläche. Die Berechnung kann durch ein Berechnungsmodul ausgeführt werden, das einen auf einer optischen Triangulationsberechnung basierenden Algorithmus umfasst.

Der Prozessor 25 umfasst Schnittstellen 25i, 25ii. Bei den Eingangsgrößen, die über die Eingangsschnittstellen 25i, 25ii eingelesen werden, handelt es sich insbesondere um die digitalen Bilddatensätze, die mit der Kamera 14 am C-Bogen 10 und mit der Kamera 24 an der Visualisierungsbrille 20 erfasst worden sind.

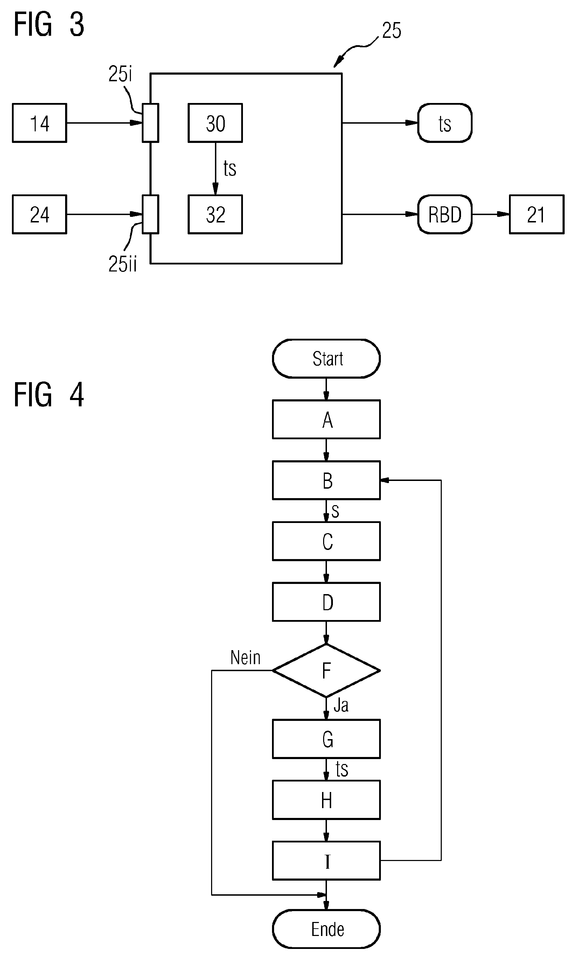

In **Figur 3** sind die Bauteile des Prozessors 25 auf schematische Weise näher gekennzeichnet. Der jeweilige Bilddatensatz, der mit der Kamera 14 erfasst worden ist, wird über die Prozessoreingangsschnittstelle 25i dem Prozessor 25 zugeführt. Der digitale Bilddatensatz, der mittels der Visualisierungsbrillenkamera 24 erfasst worden ist, wird über die Prozessor-eingangsschnittstelle 25ii dem Prozessor 25 zugeführt. Wie vorstehend bereits erläutert, ist es auch möglich, nur eine Kamera als Kamerasystem vorzusehen. In diesem Fall muss die eine Kamera dann sowohl das projizierte erste Lichtmuster des C-Bogen-Projektionsmoduls 16 als auch das projizierte zweite Lichtmuster des HMD-Projektionsmoduls 22 erfassen. In diesen Fällen werden die erfassten Bilddaten über eine gemeinsame oder über unterschiedliche Schnittstellen dem Prozessor 25 zugeleitet, der die erfassten Bilddaten dann weiterverarbeitet. Dazu umfasst der Prozessor 25, wie näher in Figur 3 dargestellt, eine Synchronisierungseinrichtung 30 und eine Fusionseinheit 32. Die Synchronisierungseinrichtung 30 ist dazu bestimmt, ein Triggersignal ts auszugeben, falls automatisch erfasst werden kann, dass der von dem C-Bogen-Projektionsmodul 16 projizierte erste ausgewählte Bereich mit dem vom HMD-Projektionsmodul 22 projizierte zweite ausgewählte Bereich übereinstimmen. Mit anderen Worten synchronisiert die Synchronisierungseinrichtung 30 den C-Bogen 10 und die Visualisierungsbrille 20 über die applizierten ersten und zweiten Lichtmuster. Wesentlich ist, dass das erste Lichtmuster beziehungsweise der erste ausgewählte Bereich von den C-Bogen-Projektionsmodul 16 abgestrahlt wird und das zweite Lichtmuster beziehungsweise der zweite ausgewählte Bereich von einem anderen Projektionsmodul, nämlich von dem HMD-Projektionsmodul 22 (der Visualisierungsbrille 20)bestrahlt wird. Der Zeitraum, in dem das C-Bogen-Projektionsmodul 16 ein Lichtmuster projiziert und der Zeitraum, in dem das HMD-Projektionsmodul 22 ein Lichtmuster appliziert müssen nicht zwangsläufig übereinstimmen. Vorzugsweise weisen die beiden Zeiträume jedoch einen Überlappungsbereich auf.

Der Prozessor 25 umfasst die Fusionseinheit 32. Die Fusionseinheit 32 ist dazu bestimmt, einen kombinierten Bilddatensatz kBD zu erzeugen, falls sie von der Synchronisierungseinheit 30 ein Triggersignal ts empfangen hat. Dies soll sicherstellen, dass die Fusionseinheit 32 nur dann aktiviert wird, falls dies auch notwendig ist und insbesondere, falls der Arzt mit seiner Blickfeldbrille 20 auch genau exakt den Bereich betrachtet, in dem der C-Bogen 10 Röntgenbilder beziehungsweise röntgenbasierte Bilder (z. B. Schichtbilder) erstellt. Wie in Figur 3 schematisch angedeutet, kann das Triggersignal ts auch an weitere Module weitergeleitet werden, die sich extern und außerhalb des Prozessors 25 befinden. Darüber hinaus kann das Triggersignal ts auch an Module weitergeleitet werden, die sich außerhalb der Visualisierungsbrille 20 befinden. Üblicherweise wird das Triggersignal ts jedoch zumindest an die Fusionseinheit 32 weitergeleitet, um diese darüber zu informieren, dass der kombinierte Bilddatensatz kBD generiert werden soll. Der kombinierte Bilddatensatz kBD wird dann auf die beiden Brillengläser 21 projiziert.

Der kombinierte Bilddatensatz kBD umfasst somit sowohl das von dem C-Bogen 10 erfasste Röntgenbild in einem Bereich des Patienten und das 3-dimensionale Oberflächenbild an der jeweiligen Körperoberfläche des Patienten. Dabei sind die beiden Bilddatensätze korreliert. Dies bedeutet, dass der Körperoberflächenbilddatensatz und der Tiefenbilddatensatz in räumlicher Beziehung aufeinander abgeglichen sind. Damit ist sichergestellt, dass das jeweilige Tiefenbild und das Körperoberflächenbild von exakt derselben Position stammen. Damit entsteht der wesentliche, vorteilhafte technische Effekt, dass der Arzt, während er den Patienten untersucht und/oder operiert auf einen Blick und ohne den Kopf abwenden zu müssen (um beispielsweise auf einen separaten Monitor zu sehen) Informationen sowohl über die Körperoberfläche als auch über Gegebenheiten erhält, die sich in der Körpertiefe der jeweils zu behandelnden Körperstruktur befinden. Darüber hinaus erhält er gleichzeitig auch noch eine dritte Bildinformation, nämlich den betrachteten Körperbereich, den er gerade im Blickfeld hat. Letzteren würde er auch sehen, wenn er ohne weitere technische Zusatzinstrumente (insbesondere ohne Visualisierungsbrille und ohne Röntgen) auf den Patienten blicken würde.

Grundsätzlich erhält der Arzt gleichzeitig drei Bildinformationen zu ein und demselben Körperbereich:
1.Körpertiefeninformationen, z. B. mittels des Röngtgenbildes,
2.Körperoberflächeninformationen über die 3-dimensionale Formen der Oberfläche, erfasst mittels des Kamerasystems 14, 24 und
3.das native Blickfeld durch Betrachten des Arztes.

Damit kann der Arzt in kürzester Zeit die maximale Bildinformation erhalten, um beispielsweise eine Schraube zur Verschraubung eines Implantates an der richtigen Stelle auf der Körperoberfläche und derart ansetzten zu können, dass die Schraube auch intern an der richtigen Stelle und im richtigen Winkel das Implantat trifft. Die letztere Information erhält der Arzt über die Röntgenbildinformationen. Diese Informationen sind insbesondere dann sehr wesentlich und vorteilhaft, falls es sich um in minimal invasive Eingriffe handelt, bei denen der Arzt am ungeöffneten Patienten einen Eingriff vornehmen soll. Dies bedeutet im chirurgischen Verfahren insbesondere einen Zeit- und einen Qualitätsvorteil, da Fehlpositionierungen von Instrumenten und/oder Werkzeugen vermieden werden können.

Im Folgenden wird ein möglicher Ablauf eines erfindungsgemäßen Visualisierungsverfahrens unter Bezugnahme auf **Figur 4** näher beschrieben:
Nach dem Start des Systems erfolgt in **Schritt A** ein Röntgenbild erfasst. Dazu kann im Vorfeld ein interessierender Bereich (ROI) ausgewählt werden.

In **Schritt B** erfolgt ein erstes Projizieren von strukturiertem Licht mittels des C-Bogens-Projektionsmoduls 16.

Nach Erfassen eines Startsignals s erfolgt in **Schritt C** ein zweites Projizieren von strukturiertem Licht mittels des HMD-Projektionsmoduls 22, das sich an der Visualisierungsbrille 20 befindet.

In einer bevorzugten Ausführungsform steuert der Prozessor 25 die Applikation des ersten Lichtmusters in Schritt B und die Applikation des zweiten Lichtmusters in Schritt C. Üblicherweise wird dasselbe Lichtmuster projiziert. Alternative Ausführungsformen können hier jedoch abweichende Steuermaßnahmen vorsehen, so dass das C-Bogen-Projektionsmodul 16 und das HMD-Projektionsmodul 22 unterschiedliche Formen von strukturiertem Licht (unterschiedliche Muster, unterschiedliche Farben etc.) ausgeben.

In **Schritt D** erfolgt das Erfassen des digitalen Bilddatensatzes. Der digitale Bilddatensatz umfasst die mit dem Kamerasystem 14, 24 erfassten Bilddaten der jeweiligen Körperoberfläche. Der digitale Bilddatensatz kann vorzugsweise in dem Prozessor 25 berechnet werden. Der digitale Bilddatensatz bezieht sich vorzugsweise auf eine 3-dimensionale Oberflächenstruktur des projizierten Bereichs. Der digitale Bilddatensatz kennzeichnet sich in einem weiteren Aspekt dadurch, dass er keine Tiefeninformation enthält. Dies bedeutet, dass der digitale Bilddatensatz nur Bilddaten von der Körperoberfläche umfasst und keine Bilddaten, die das Körperinnere betreffen.

Im **Schritt F** erfolgt eine Fallunterscheidung. In diesem Schritt wird geprüft, ob der erste projizierte Bereich mit dem zweiten projizierten Bereich identisch übereinstimmen. Dies dient insbesondere zur Synchronisierung des C-Bogens 10 mit der Visualisierungsbrille 20. Falls die Bereiche nicht übereinstimmen, kann davon ausgegangen werden, dass der Arzt mit seiner Visualisierungsbrille 20 auf einen anderen Bereich blickt als der Bereich, der von dem Röntgengerät 10 bestrahlt wird und von dem die Tiefenbildinformationen erfasst werden. In diesem Fall kann nochmals zu Schritt B verzweigt werden, so dass nochmals die Projektion der beiden Lichtmuster aktiviert wird.

Andernfalls, falls also der erste projizierte Bereich mit dem zweiten projizierten Bereich übereinstimmt, erfolgt in **Schritt G** die Ausgabe des Triggersignals ts.

In **Schritt H** wird ein kombinierter Bilddatensatz kBD erzeugt. Der kombinierte Bilddatensatz umfasst die Tiefenbildinformationen und die (3-dimensionalen) Körperoberflächeninformationen. Beide Bilddatensätze sind ortsaufgelöste gematched und werden zu einem Fusionsbild verschmolzen.

In **Schritt I** erfolgt das Ausgeben des berechneten kombinierten Bilddatensatzes kBD auf der Visualisierungsbrille 20 und insbesondere auf den beiden Brillengläsern 21 derselben.

Wie in Figur 4 dargestellt, kann daraufhin das Verfahren nochmals iterativ ausgeführt werden, indem die beiden Projektionsmodule nochmals strukturiertes Licht projizieren und das Verfahren erneut ausgeführt wird. Andernfalls, und insbesondere falls ausreichend Informationen bereitgestellt worden sind, kann das Verfahren auch enden.

Wesentlich für das erfindungsgemäße Visualisierungssystem und -verfahren ist, dass der Arzt bei Aktivieren der Visualisierungsfunktion immer einen automatisch aktualisiert berechneten kombinierten Bilddatensatz kBD erhält, der automatisch mit seiner Blickfeldeinrichtung beziehungsweise mit seinem Blickfeld übereinstimmt. Ohne den Kopf und den Blick abwenden zu müssen, kann er gleichzeitig Körperoberflächeninformationen und Körpertiefeninformationen abrufen.

Während der Schwenk- beziehungsweise Drehbewegung des C-Bogens 10 verändert sich das Lichtmuster, das von den C-Bogen-Projektionsmodul 16 projiziert worden ist. Des Weiteren ändert sich die Form des projizierten Lichtes durch die unterschiedliche 3-dimensionale Körperoberfläche des Patienten, der sich auf dem Operationstisch befindet. Diese optischen Daten werden mittels eines optischen Triangulationsverfahrens in eine virtuelle Körperoberfläche des Patienten transferiert. Da die 3-dimensionalen Röntgendaten und die durch das Kamerasystem 14, 24 erfassten Körperoberflächendaten korreliert sind, was durch die feste Anordnung der jeweiligen Projektionsmodule 16, 22 und der Kameras 14, 24 möglich ist, kann der Arzt den Eindruck erhalten, dass er bei Aktivierung der erfindungsgemäßen Visualisierung in seinem Blickfeld einen quasi transparenten Eindruck von der Körperfläche erhält und somit durch die Körperoberfläche hindurch sehen kann.

Auf der Visualisierungsbrille 20 werden jeweils immer nur die aktuellen Bilddaten in dem kombinierten Bilddatensatz kBD visualisiert und angezeigt. Somit kann sichergestellt werden, dass der Arzt immer genau die Zusatzinformationen (Körperoberflächeninformationen, Körpertiefeninformationen) erhält, die mit dem Bereich übereinstimmen, auf den er aktuell gerade blickt. Des Weiteren kann durch die Anwendung des Startsignals s und des Triggersignals ts erreicht werden, dass der Arzt nicht mit unnötigen Zusatzinformationen belästigt wird. Falls kein Aktivierungssignal s, ts erfasst werden kann, wird kein Bilddatensatz und insbesondere kein kombinierter Bilddatensatz kBD auf den Brillengläsern 21 der Visualisierungsbrille 20 dargestellt. In diesem Fall funktioniert die Visualisierungsbrille 20 nicht als Projektionsmittel sondern der Arzt erhält dasselbe Sehergebnis im Vergleich zu der Situation, wenn er ohne Visualisierungsbrille 20 arbeiten würde.

Falls das Visualisierungssystem aktiviert ist und der Arzt zusätzliche Bildinformationen (Tiefeninformationen, Oberflächeninformationen) erhalten möchte, so erhält er diese stets in aktualisierter Form, und insbesondere auch dann, falls er seinen Kopf bewegt und einen anderen Bereich auf der Körperoberfläche des Patienten betrachtet. Dies hat die Wirkung, dass der Arzt mittels der Visualisierungsbrille 20 in der Art einer Lupe quasi "durch" genau den Bereich des Körpers sehen kann, den er gerade betrachtet.

Gemäß einem Aspekt erfolgt das Erfassen der Tiefeninformation (mittels der bildgebenden Einrichtung 10), und das Erfassen in der Körperoberflächeninformation (mittels des Kamerasystems 14, 24) über die Projektion von strukturiertem Licht gleichzeitig. Dies bedeutet, dass während die bildgebende Einrichtung 10 Röntgenbilder erfasst auch die beiden Projektionsmodule 16, 22 aktiviert sind, um das strukturierte Licht in dem ausgewählten ersten und zweiten Bereich zu applizieren.

Vorstehend wurde von einem "ausgewählten ersten/zweiten Bereich" gesprochen, da es in einer Konfigurationsphase möglich ist, das strukturierte Licht nur auf einen jeweils interessanten Körperbereich zu projizieren. Dies ermöglicht es, dass nicht unnötige Bildoberflächendaten gesammelt werden müssen. In der Regel ist nur der um den operativen Eingriff herum befindliche Bereich von Interesse, so dass es genügt, die Projektionsmodule so einzustellen, dass sie nur einen ausgewählten Bereich beleuchten. Gemäß einer alternativen Ausführungsform ist es voreingestellt, dass der ausgewählte erste/zweite Bereich aus dem vollständigen ersten/zweiten Bereich gebildet wird.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Für einen Fachmann ist es insbesondere offensichtlich, dass die Erfindung teilweise oder vollständig in Soft- und/oder Hardware und/oder auf mehrere physikalische Produkte - dabei insbesondere auch Computerprogrammprodukte - verteilt realisiert werden kann.

### Bezugszeichenliste

- 10: Bildgebende Einrichtung, insbesondere C-Bogen-Röntgengerät
- 12: Röntgenquelle
- 14: Kamera im C-Bogen
- 16: C-Bogen-Projektionsmodul
- 18: Rollwagen zur Lagerung des C-Bogens
- 19: Recheneinheit bzw. Rechner
- 20: Visualisierungsbrille
- 22: HMD-Projektionsmodul
- 24: Kamera in der Visualisierungsbrille
- 25: Prozessor
- 21: Brillengläser
- 25i: Prozessor-Eingangsschnittstelle
- 25ii: weitere Prozessor-Eingangsschnittstelle
- 30: Synchronisierungseinrichtung
- 32: Fusionseinheit
- ts: Triggersignal
- kBD: kombinierter Bilddatensatz
- s: Startsignal
- A: Erfasser Röntgenbild
- B: Erstes Projizieren von erstem Lichtmuster mit C-Bogen-Projektionsmodul 16
- C: Zweites Projizieren von zweitem Lichtmuster mit HMD-Projektionsmodul 22
- D: Erfasse digitalen Bilddatensatz
- F: Automatische Überprüfung: Stimmt erstes projiziertes Lichtmuster mit zweitem projiziertem Lichtmuster identisch überein?
- G: Ausgabe Triggersignal ts
- H: Erzeuge kombinierten Bilddatensatz kBD
- I: Ausgeben des kombinierten Bilddatensatzes kBD auf der Visualisierungsbrille 20

## Patentansprüche

1. Intraoperatives markerloses Visualisierungssystem zur Visualisierung von dreidimensionalen Strukturen im Rahmen einer Intervention an einem Patienten mittels einer sich während einer Bildakquisition bewegenden, bildgebenden Einrichtung (10), umfassend:
- Ein C-Bogen-Projektionsmodul (16), das dazu bestimmt ist, strukturiertes Licht auf einen ersten ausgewählten Bereich einer Körperoberfläche des Patienten zu projizieren, wobei das C-Bogen-Projektionsmodul (16) in der bildgebenden Einrichtung (10) integriert ist und während sich die bildgebende Einrichtung (10) zur Bildakquisition bewegt
- Eine Visualisierungsbrille (20), die ein HMD-Projektionsmodul (22) umfasst, das dazu bestimmt ist, auf ein Startsignal (s) hin strukturiertes Licht auf einen zweiten ausgewählten Bereich einer Körperoberfläche des Patienten zu projizieren
- Ein Kamerasystem (14, 24), das dazu bestimmt ist, das von dem C-Bogen-Projektionsmodul (16) und das von dem HMD-Projektionsmodul (22) projizierte strukturierte Licht zu erfassen, um mit einem Prozessor (25) zumindest einen digitalen Bilddatensatz einer 3D-Oberflächenstruktur zu berechnen, und wobei das Kamerasystem (14, 24) zumindest teilweise in der bildgebenden Einrichtung (10) und/oder in der Visualisierungsbrille (20) integriert ist
- Eine Synchronisierungseinrichtung (30), die dazu bestimmt ist, ein Triggersignal (ts) auszugeben, wenn sie automatisch erfasst, dass der vom C-Bogen-Projektionsmodul (16) projizierte erste ausgewählte Bereich mit dem vom HMD-Projektionsmodul (22) projizierte zweite ausgewählte Bereich übereinstimmen
- Eine Fusionseinheit (32), die dazu bestimmt ist, in Antwort auf das Triggersignal (ts) der Synchronisierungseinheit (30), einen kombinierten Bilddatensatz (kBD) zu erzeugen und auf der Visualisierungsbrille (20) auszugeben, wobei der kombinierte Bilddatensatz (kBD) das von der bildgebenden Einrichtung (10) erfasste Bild und den digitalen Bilddatensatz des Kamerasystems (14, 24) der Körperoberfläche korreliert.

2. Intraoperatives Visualisierungssystem nach Anspruch 1, bei dem die Synchronisierungseinrichtung (30) und/oder die Fusionseinheit (32) in die Visualisierungsbrille (20) integriert ist/sind.

3. Intraoperatives Visualisierungssystem nach Anspruch 1, bei dem die Synchronisierungseinrichtung (30) und/oder die Fusionseinheit (32) außerhalb der Visualisierungsbrille (20) angeordnet ist/sind und mit ihr in Datenaustausch steht/stehen.

4. Intraoperatives Visualisierungssystem nach Anspruch 1, bei dem die Synchronisierungseinrichtung (30) und die Fusionseinheit (32) in einem Modul integriert sind.

5. Intraoperatives Visualisierungssystem nach einem der vorstehenden Ansprüche, bei dem der Prozessor (25) den vom Kamerasystem (14, 24) erfassten digitalen Bilddatensatz auch während einer Bewegung, insbesondere einer rotatorischen und/oder translatorischen Bewegung der bildgebenden Einrichtung (10), und/oder während einer Bewegung des HMD-Projektionsmoduls (22) der Visualisierungsbrille (20) berechnet, indem ein automatisches, computer-basiertes Verfahren zur optischen Triangulation angewendet wird.

6. Intraoperatives Visualisierungssystem nach einem der vorstehenden Ansprüche, bei dem die bildgebende Einrichtung (10) zur Bilddatenakquisition von Strukturen bestimmt ist, die sich nicht auf einer Oberfläche des Patienten, sondern im Inneren des Patienten befinden.

7. Intraoperatives Visualisierungssystem nach einem der vorstehenden Ansprüche, bei dem das Kamerasystem (14, 24) zur Erfassung von dreidimensionalen Oberflächenstrukturen bestimmt ist, die sich nicht im Inneren des Patienten, sondern auf einer Oberfläche des Patienten befinden.

8. Intraoperatives markerloses Visualisierungsverfahren zur Visualisierung von dreidimensionalen Strukturen im Rahmen einer Intervention an einem Patienten mittels einer sich während einer Bildakquisition bewegenden, bildgebenden Einrichtung (10), umfassend folgende Verfahrensschritte:
- Erfassen (A) eines Bilddatensatzes mit der bildgebenden Einrichtung (10)
- Erstes Projizieren (B) von strukturiertem Licht auf einen ersten ausgewählten Bereich einer Körperoberfläche des Patienten mit einem C-Bogen-Projektionsmodul (16), das in der bildgebenden Einrichtung (10) integriert ist, während sich die bildgebende Einrichtung (10) zum Zwecke der Bildakquisition bewegt
- Zweites Projizieren (C) von strukturiertem Licht auf einen zweiten ausgewählten Bereich der Körperoberfläche des Patienten mit einem HMD-Projektionsmodul (22), das in einer Visualisierungsbrille (20) integriert ist,
- Erfassen zumindest eines digitalen Bilddatensatzes (D), umfassend dem projizierten ersten und/oder zweiten ausgewählten Bereich zum Berechnen einer 3D-Oberflächenstruktur in dem jeweils projizierten Bereich
- Ausgeben (G) eines Triggersignals (ts), falls automatisch erfasst wird, dass der erste ausgewählte Bereich und der zweite ausgewählten Bereich übereinstimmen
- In Antwort auf das Triggersignal (ts): Erzeugen (H) eines kombinierten Bilddatensatzes (kBD) und Ausgeben desselben auf der Visualisierungsbrille (20), wobei der kombinierte Bilddatensatz (kBD) berechnet wird, indem das von der bildgebenden Einrichtung (10) erfasste Bild mit dem zumindest einen digitalen Bilddatensatz mit der berechneten 3D-Oberflächenstruktur korreliert wird.

9. Intraoperatives markerloses Visualisierungsverfahren gemäß vorstehendem Verfahrensanspruch, bei dem das erste Projizieren (B) und das zweite Projizieren (C) zeitgleich oder in einem übereinstimmenden Zeitbereich ausgeführt werden.

10. Intraoperatives markerloses Visualisierungsverfahren gemäß einem der vorstehenden Verfahrensansprüche, bei dem das Erfassen (D) des zumindest einen digitalen Bilddatensatzes ausgeführt wird, wenn das zweite Projizieren (C) ausgeführt wird.

11. Intraoperatives markerloses Visualisierungsverfahren gemäß einem der vorstehenden Verfahrensansprüche, bei dem das Erfassen (D) des zumindest einen digitalen Bilddatensatzes zeitgleich oder in einem übereinstimmenden Zeitbereich ausgeführt wird wie die Bildakquisition mittels der bildgebenden Einrichtung (10).

12. Computerprogramm ladbar oder geladen in einen Speicher eines Computers oder Prozessors mit von dem Computer oder Prozessor lesbaren Befehlen zur Ausführung des Verfahrens nach den vorstehenden Verfahrensansprüchen, wenn die Befehle auf dem Computer oder Prozessor ausgeführt werden.

## Claims

1. Intraoperative marker-less visualization system for visualizing three-dimensional structures during an intervention on a patient by way of an imaging device (10) that moves during image acquisition, comprising:
- a C-arm projection module (16) which is intended for projecting structured light onto a first selected region of a body surface of the patient, wherein the C-arm projection module (16) is integrated in the imaging device (10) and while the imaging device (10) moves for image acquisition
- visualization spectacles (20) which comprise a HMD projection module (22) that is intended for projecting, upon a start signal (s), structured light onto a second selected region of a body surface of the patient
- a camera system (14, 24) which is intended for capturing the structured light projected by the C-arm projection module (16) and by the HMD projection module (22) so as to compute at least one digital image data set of a 3D surface structure using a processor (25), and wherein the camera system (14, 24) is at least partly integrated in the imaging device (10) and/or in the visualization spectacles (20)
- a synchronization device (30) which is intended for outputting a trigger signal (ts) once it automatically detects that the first selected region projected by the C-arm projection module (16) matches the second selected region projected by the HMD projection module (22),
- a fusion unit (32) which is intended for generating, in response to the trigger signal (ts) of the synchronization unit (30), a combined image data set (kBD) and to output it on the visualization spectacles (20), wherein the combined image data set (kBD) correlates the image captured by the imaging device (10) and the digital image data set of the camera system (14, 24) of the body surface.

2. Intraoperative visualization system according to Claim 1, in which the synchronization device (30) and/or the fusion unit (32) is/are integrated in the visualization spectacles (20).

3. Intraoperative visualization system according to Claim 1, in which the synchronization device (30) and/or the fusion unit (32) is/are arranged outside the visualization spectacles (20) and is/are in data communication therewith.

4. Intraoperative visualization system according to Claim 1, in which the synchronization device (30) and the fusion unit (32) are integrated in one module.

5. Intraoperative visualization system according to one of the preceding claims, in which the processor (25) also computes the digital image data set captured by the camera system (14, 24) during a movement, in particular a rotational and/or translational movement of the imaging device (10), and/or during a movement of the HMD projection module (22) of the visualization spectacles (20) by applying an automatic, computer-based method for optical triangulation.

6. Intraoperative visualization system according to one of the preceding claims, in which the imaging device (10) is intended for image data acquisition of structures which are not located on a surface of the patient but inside the patient.

7. Intraoperative visualization system according to one of the preceding claims, in which the camera system (14, 24) is intended for capturing three-dimensional surface structures which are not located inside the patient but on a surface of the patient.

8. Intraoperative marker-less visualization method for visualizing three-dimensional structures during an intervention on a patient by way of an imaging device (10) that moves during image acquisition, comprising the following method steps:
- capturing (A) an image data set with the imaging device (10)
- first projection (B) of structured light onto a first selected region of a body surface of the patient with a C-arm projection module (16) that is integrated in the imaging device (10) while the imaging device (10) moves for the purpose of image acquisition
- second projection (C) of structured light onto a second selected region of the body surface of the patient with a HMD projection module (22) that is integrated in a pair of visualization spectacles (20),
- capturing at least one digital image data set (D), comprising the projected first and/or second selected region for computing a 3D surface structure in the respectively projected region
- outputting (G) a trigger signal (ts) if it is automatically ascertained that the first selected region and the second selected region match
- in response to the trigger signal (ts): generating (H) a combined image data set (kBD) and outputting same on the visualization spectacles (20), wherein the combined image data set (kBD) is computed by correlating the image captured by the imaging device (10) with the at least one digital image data set with the computed 3D surface structure.

9. Intraoperative marker-less visualization method according to the preceding method claim, in which the first projection (B) and the second projection (C) are carried out at the same time or within a matching timeframe.

10. Intraoperative marker-less visualization method according to one of the preceding method claims, in which the capturing (D) of the at least one digital image data set is carried out when the second projection (C) is carried out.

11. Intraoperative marker-less visualization method according to one of the preceding method claims, in which the capturing (D) of the at least one digital image data set is carried out at the same time or within a matching timeframe as the image acquisition using the imaging device (10).

12. Computer program, which is or can be loaded onto a memory of a computer or processor with commands that are readable by the computer or processor for carrying out the method according to the preceding method claims if the commands are carried out on the computer or processor.

## Revendications

1. Système de visualisation sans marqueur peropératoire pour la visualisation de structures tridimensionnelles dans le cadre d'une intervention sur un patient au moyen d'un dispositif d'imagerie (10) se déplaçant pendant une acquisition d'images, comprenant:
- un module de projection arqué en C (16), qui est destiné à projeter une lumière structurée sur une première région sélectionnée d'une surface du corps du patient, dans lequel le module de projection arqué en C (16) est intégré dans le dispositif d'imagerie (10) et pendant que le dispositif d'imagerie (10) se déplace pour l'acquisition d'images,
- des lunettes de visualisation (20), qui comprennent un module de projection HMD (22), qui est destiné à projeter, sous un signal de démarrage (s), une lumière structurée sur une deuxième région sélectionnée d'une surface du corps du patient,
- un système de caméra (14, 24), qui est destiné à détecter la lumière structurée projetée par le module de projection arqué en C (16) et par le module de projection HMD (22), afin de calculer avec un processeur (25) au moins un jeu de données d'images numériques d'une structure de surface 3D, et dans lequel le système de caméra (14, 24) est au moins en partie intégré dans le dispositif d'imagerie (10) et/ou dans les lunettes de visualisation (20),
- un dispositif de synchronisation (30), qui est destiné à émettre un signal de déclenchement (ts), lorsqu'il détecte automatiquement que la première région sélectionnée éclairée par le module de projection arqué en C (16) coïncide avec la deuxième région sélectionnée éclairée par le module de projection HMD (22),
- une unité de fusion (32), qui est destinée, en réponse au signal de déclenchement (ts) de l'unité de synchronisation (30), à produire un jeu de données d'images combiné (kBD) et à l'envoyer sur les lunettes de visualisation (20), dans lequel le jeu de données d'images combiné (kBD) corrèle l'image détectée par le dispositif d'imagerie (10) et le jeu de données d'images numériques du système de caméra (14, 24) de la surface du corps.

2. Système de visualisation peropératoire selon la revendication 1, dans lequel le dispositif de synchronisation (30) et/ou l'unité de fusion (32) est/sont intégrés dans les lunettes de visualisation (20).

3. Système de visualisation peropératoire selon la revendication 1, dans lequel le dispositif de synchronisation (30) et/ou l'unité de fusion (32) est/sont disposés à l'extérieur des lunettes de visualisation (20) et est/sont en relation d'échange de données avec celles-ci.

4. Système de visualisation peropératoire selon la revendication 1, dans lequel le dispositif de synchronisation (30) et l'unité de fusion (32) sont intégrés dans un module.

5. Système de visualisation peropératoire selon l'une quelconque des revendications précédentes, dans lequel le processeur (25) calcule le jeu de données d'images numériques détectées par le système de caméra (14, 24) aussi pendant un mouvement, en particulier un mouvement de rotation et/ou de translation du dispositif d'imagerie (10), et/ou pendant un mouvement du module de projection HMD (22) des lunettes de visualisation (20), en utilisant un procédé automatique de triangulation optique assisté par ordinateur.

6. Système de visualisation peropératoire selon l'une quelconque des revendications précédentes, dans lequel le dispositif d'imagerie (10) est destiné à l'acquisition de données d'images de structures qui ne se trouvent pas sur une surface du patient, mais à l'intérieur du patient.

7. Système de visualisation peropératoire selon l'une quelconque des revendications précédentes, dans lequel le système de caméra (14, 24) est destiné à la détection de structures de surface tridimensionnelles, qui ne se trouvent pas à l'intérieur du patient mais sur une surface du patient.

8. Procédé de visualisation sans marqueur peropératoire pour la visualisation de structures tridimensionnelles dans le cadre d'une intervention sur un patient au moyen d'un dispositif d'imagerie (10) se déplaçant pendant une acquisition d'images, comprenant les étapes suivantes:
- détection (A) d'un jeu de données d'images avec le dispositif d'imagerie (10);
- première projection (B) de lumière structurée sur une première région sélectionnée d'une surface du corps du patient avec un module de projection arqué en C (16), qui est intégré dans le dispositif d'imagerie (10) pendant que le dispositif d'imagerie (10) se déplace pour l'acquisition d'images,
- deuxième projection (C) de lumière structurée sur une deuxième région sélectionnée de la surface du corps du patient avec un module de projection HMD (22), qui est intégré dans des lunettes de visualisation (20),
- détection d'au moins un jeu de données d'images numériques (D) comprenant la première et/ou la deuxième région sélectionnée éclairée pour le calcul d'une structure de surface 3D dans la région respectivement éclairée,
- émission (G) d'un signal de déclenchement (ts), dans le cas où l'on détecte automatiquement que la première région sélectionnée et la deuxième région sélectionnée coïncident,
- en réponse au signal de déclenchement (ts): production (H) d'un jeu de données d'images combiné (kBD) et envoi de celui-ci sur les lunettes de visualisation (20), dans lequel on calcule le jeu de données d'images combiné (kBD), en corrélant l'image détectée par le dispositif d'imagerie (10) avec ledit au moins un jeu de données d'images numériques avec la structure de surface 3D calculée.

9. Procédé de visualisation sans marqueur peropératoire selon la revendication de procédé précédente, dans lequel on effectue la première projection (B) et la deuxième projection (C) simultanément ou dans une plage temporelle correspondante.

10. Procédé de visualisation sans marqueur peropératoire selon l'une quelconque des revendications de procédé précédentes, dans lequel on effectue la détection (D) dudit au moins un jeu de données d'images numériques lorsque l'on effectue la deuxième projection (C).

11. Procédé de visualisation sans marqueur peropératoire selon l'une quelconque des revendications de procédé précédentes, dans lequel on effectue la détection (D) dudit au moins un jeu de données d'images numériques simultanément à l'acquisition d'images au moyen du dispositif d'imagerie (10) ou dans une plage temporelle correspondante.

12. Programme informatique pouvant être ou étant chargé dans une mémoire d'un ordinateur ou d'un processeur avec des instructions lisibles par l'ordinateur ou le processeur pour l'exécution du procédé selon les revendications de procédé précédentes, lorsque les instructions sont exécutées sur l'ordinateur ou le processeur.
